Europäisches Patentamt

European Patent Office

Office européen des brevets

(19)

(11) Veröffentlichungsnummer : **0 350 738 B1**

(12) **EUROPÄISCHE PATENTSCHRIFT**

(45) Veröffentlichungstag der Patentschrift :
**14.10.92 Patentblatt 92/42**

(51) Int. Cl.⁵ : **A61K 31/275**

(21) Anmeldenummer : **89112045.3**

(22) Anmeldetag : **01.07.89**

(54) **(S)-Emopamil zur Anwendung bei der Behandlung von Migräne.**

(30) Priorität : **09.07.88 DE 3823378**

(43) Veröffentlichungstag der Anmeldung :
**17.01.90 Patentblatt 90/03**

(45) Bekanntmachung des Hinweises auf die
Patenterteilung :
**14.10.92 Patentblatt 92/42**

(84) Benannte Vertragsstaaten :
**AT BE CH DE FR GB IT LI NL SE**

(56) Entgegenhaltungen :
**EP-A- 0 147 707**
**EP-A- 0 329 010**
**DRUGS OF TODAY, Band 24, Nr. 2, Februar**
**1988; D.A.GREENBERG, Seiten 133-142.**
**ARZNEIMITTEL-FORSCHUNG, Band 39(I), Nr.**
**3, März 1989; L.SZABO, Seiten 309-314.**
**ARZNEIMITTEL-FORSCHUNG, Band 39(I), Nr.**
**3, März 1989; L.SZABO et al., Seiten 314-319.**

(56) Entgegenhaltungen :
**DRUGS, Band 32, 1987, ADIS Press Ltd;**
**K.J.TIETZE et al., Seiten, 531-538.**
**TRENDS PHARMACOL. SCI., Band 10, Nr. 6,**
**Juni 1989; F.V.DEFEUDIS, Seiten 215-217.**
**NEUROLOGY, Band 34, Juli 1984; H.G.MAR-**
**KLEY et al., Seiten 973-976.**

(73) Patentinhaber : **KNOLL AG**
**Knollstrasse 32**
**W-6700 Ludwigshafen (DE)**

(72) Erfinder : **Baldinger, Verena, Dr.**
**Schiffsgasse 6**
**W-6900 Heidelberg (DE)**
Erfinder : **Unger, Liliane, Dr.**
**Waltraudenstrasse 14**
**W-6700 Ludwigshafen (DE)**
Erfinder : **Szabo, Laszlo, Dr.**
**Kastellweg 10**
**W-6900 Heidelberg (DE)**

(74) Vertreter : **Karau, Wolfgang, Dr. et al**
**BASF Aktiengesellschaft Carl-Bosch-Strasse**
**38**
**W-6700 Ludwigshafen (DE)**

EP 0 350 738 B1

## Beschreibung

Die vorliegende Erfindung betrifft die Verwendung von (S)-Emopamil bei der vorbeugenden und akuten Behandlung von Migräne.

In EP-OS 147 707 ist das 2-Isopropyl-5-(methylphenethylamino)-2-phenylvaleronitril (im folgenden als Emopamil bezeichnet) und seine Enantiomeren sowie deren Verwendung bei der Bekämpfung von Krankheiten u.a. bei Sauerstoffmangel-Zuständen des Gehirns beschrieben.

Es ist bekannt, daß Verapamil außer seiner calciumantagonistischen zusätzlich eine serotoninantagonistische Wirkung aufweist, die jedoch im Vergleich zu bekannten Serotoninantagonisten schwach ausgeprägt ist [J. E. Taylor u. F. V. DeFeudis, Eur. J. Pharmacol. 106, 215-216 (1985)]. Weiterhin ist bekannt, daß Serotonin eine große Bedeutung in der Pathogenese der Migräne hat [D. S. Houston u. P. M. Vanhoutte, Drugs 31, 149-163 (1986); G. Johnson, Ann. Rep. Med. Chem. 22, 41-50 (1987)]. Es ist anzunehmen, daß sich Calcium- und Serotoninantagonismus bei der Migräne additiv, wenn nicht sogar potenzierend auswirken.

Klinische Untersuchungen haben gezeigt, daß der prophylaktische Einsatz des Calciumantagonisten Verapamil eine wirksame Therapie von klassischer und atypischer Migräne sowie Cluster-Kopfschmerzen darstellt [D. A. Greenberg, Drugs of Today 24, 133-142 (1988); K. J. Tietze u.a., Drugs 32, 531-538 (1987)].

Optimale Behandlungserfolge wurden jedoch erst nach einer Behandlungsdauer von einem Monat erzielt [G. D. Solomon u.a., JAMA 250, 2500-2502 (1983); H. G. Markley u.a., Neurology 34, 973-976 (1984)], was wahrscheinlich auf die geringe akute Hirnverfügbarkeit von Verapamil und/oder auf einen weniger stark ausgeprägten Serotoninantagonismus zurückzuführen ist.

Es wurde nun gefunden, daß (S)-Emopamil und dessen physiologisch verträglichen Salze eine Antiserotoninwirkung aufweisen, die die Wirkstärke bekannter Serotoninantagonisten erreicht und die Vergleichsverbindung Verapamil um ein Vielfaches übertrifft. Zudem konnte anhand des Index der zerebralen Aufnahme (brain uptake index, BUI) gezeigt werden, daß das Verapamilderivat (S)-Emopamil die Blut-Hirn-Schranke schneller und in wesentlich größerer Menge passiert als Verapamil.

Die überlegene serotoninantagonistische Wirkung von (S)-Emopamil läßt sich beispielsweise durch die Hemmung der durch Serotonin induzierten Blutdrucksteigerung an der despinalisierten Ratte bei intravenöser Applikation zeigen:

In Amobarbitalnarkose (120 mg/kg Körpergewicht i.p.) werden bei 200 bis 280 g schweren männlichen Sprague-Dawley-Ratten die A. carotis und die V. jugularis kanüliert, beidseitig Vagus und Sympathikus durchtrennt und die Tiere an eine Atempumpe angeschlossen. Die Despinalisierung erfolgt mittels eines Stabes durch die Orbita. Injektionen von 0,0215 mg/kg Serotonin i.v. steigern bei unvorbehandelten Kontrolltieren den arteriellen Mitteldruck von $53 \pm 0,6$ mmHg um $80 \pm 1,2$ mmHg (n = 95). Kriterium einer Substanzwirkung ist die relative Hemmung ($\delta$ %) der durch Serotonin ausgelösten Blutdrucksteigerung ($\delta$ mmHg). Die Auswertung erfolgt quantitativ als Analyse der linearen Regression (y = a + b x) zwischen log Dosis (mg/kg) und relativer Hemmung ($\delta$ %) der Serotonin-Blutdrucksteigerung. Für Vergleiche wurden als ED 50% die Dosen berechnet, welche die Serotoninblutdrucksteigerung um 50% hemmen.

Es wurden folgende Werte erhalten:

|  | ED 50% |
|---|---|
| Verapamil | 0,36 mg/kg |
| (S)-Emopamil | 0,02 mg/kg |

Die Werte zeigen, daß (S)-Emopamil an der despinalisierten Ratte nach i.v. Applikation 18 mal stärker serotoninantagonistisch wirkt als Verapamil.

Bei oraler Applikation ist (S)-Emopamil in der serotoninantagonistischen Wirkung der Vergleichsverbindung Verapamil um den Faktor 4,6 überlegen.

Die gute cerebrale Verfügbarkeit (BUI-Werte) von (S)-Emopamil im Vergleich zu Verapamil wurde mittels Injektion in die A. carotis communis der narkotisierten Ratte nach einmaliger Passage durch die Hirnkapillaren bestimmt [Methodik: W. H. Oldendorf, Brain Research 24, 372-376 (1970)]. Die [14C]-markierten Testsubstanzen wurden in mit HEPES gepufferten Ringer-Lösungen (pH=7,35) zusammen mit [3H]-markiertem Wasser als internem Standard verabreicht. Die errechneten BUI-Werte geben die zerebrale Aufnahme in Prozent der Wasseraufnahme an.

Es wurden folgende BUI-Werte ermittelt:

Verapamil        40,6 %
(S)-Emopamil     110 %

(S)-Emopamil ist aufgrund seiner hervorragenden serotoninantagonistischen Wirkung und seiner hohen zerebralen Verfügbarkeit besonders geeignet für die Prophylaxe und die Anfallsbehandlung von primären Kopfschmerzen aller Art, insbesondere von klassischer und atypischer Migräne, Cluster-Kopfschmerzen sowie ihrer Mischformen.

(S)-Emopamil kann in üblicher Weise oral, parenteral (intravenös, intramuskulär, subkutan) oder rektal verabfolgt werden.

Als physiologisch verträgliche Säuren kommen beispielsweise in Frage: Salzsäure, Bromwasserstoffsäure, Schwefelsäure, Phosphorsäure, Essigsäure, Maleinsäure, Milchsäure, Weinsäure, Zitronensäure und Fumarsäure.

Die Dosierung hängt vom Alter, Zustand und Gewicht des Patienten sowie von der Applikationsart ab. In der Regel beträgt die tägliche Wirkstoffdosis zwischen etwa 1 und 50 mg/kg Körpergewicht bei oraler und rektaler Gabe und zwischen etwa 0,1 und 5 mg/kg Körpergewicht bei parenteraler Gabe.

(S)-Emopamil kann in den gebräuchlichen galenischen Applikationsformen fest oder flüssig angewendet werden, z.B. als Tabletten, Filmtabletten, Dragees, Kapseln, Pulver, Granulate, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Suppositoriengrundmassen, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gewichtsprozent.

Die folgenden Beispiele erläutern die Erfindung.

Beispiel 1

Auf einer Tablettenpresse werden in üblicher Weise Tabletten folgender Zusammensetzung gepreßt:
40 mg     (S)-Emopamil-Hydrochlorid
120 mg     Maisstärke
13,5 mg     Gelatine
45 mg     Milchzucker
2,25     mg Aerosil® (chemisch reine Kieselsäure in submikroskopisch feiner Verteilung)
6,75 mg     Kartoffelstärke (als 6 %iger Kleister)

Beispiel 2

In üblicher Weise werden Dragees folgender Zusammensetzung hergestellt:
20 mg     (S)-Emopamil-Hydrochlorid
60 mg     Kernmasse
60 mg     Verzuckerungsmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 3

10 g (S)-Emopamil-Hydrochlorid werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

Beispiel 4

In üblicher Weise werden Suppositorien folgender Zusammensetzung hergestellt:
100 mg     (S)-Emopamil-Hydrochlorid
40 mg     Aerosil®
1900 mg     Hartfett

**Patentansprüche**

1.  Verwendung von (S)-Emopamil und dessen physiologisch verträglichen Salze zur Herstellung von Arzneimitteln zur vorbeugenden und akuten Behandlung von Migräne.

**Claims**

1.  The use of (S)-emopamil and the physiologically tolerated salts thereof for preparing drugs for the preventive and acute treatment of migraine.

**Revendications**

1.  Utilisation du (S)-Emopamil et de ses sels acceptables pour l'usage pharmaceutique pour la préparation de médicaments prévus pour la prévention et le traitement des migraines.